# EUROPEAN PATENT SPECIFICATION

(11) **EP 3 717 502 B1**
(45) Date of publication and mention of the grant of the patent: **23.04.2025**
(21) Application number: 18882759.6
(22) Date of filing: 28.11.2018
(51) Int. Cl.: C07H 21/04, C12Q 1/68, G01N 30/00, G01N 33/53, G01N 33/574, C12Q 1/6886

(54) **COMPOSITIONS AND METHODS FOR CHARACTERIZING CANCER**
ZUSAMMENSETZUNGEN UND VERFAHREN ZUR CHARAKTERISIERUNG VON KREBS
COMPOSITIONS ET PROCÉDÉS DE CARACTÉRISATION DU CANCER

(30) Priority: 29.11.2017 US 201762592009 P; 22.05.2018 US 201862674883 P
(43) Date of publication of application: 07.10.2020
(73) Proprietor: The Regents of The University of Michigan, Ann Arbor, MI 48109-2590 (US)
(72) Inventor: LERARIO, Antonio M., Ann Arbor, Michigan 48109-2590 (US); MOHAN, Dipika, Ann Arbor, Michigan 48109-2590 (US); HAMMER, Gary, Ann Arbor, Michigan 48109-2590 (US)
(74) Representative: Glawe, Delfs, Moll
(86) International application number: PCT/US2018/062709
(87) International publication number: WO 2019/108568

(56) References cited:
- US-A1- 2014 045 915
- MARIA CANDIDA B V FRAGOSO ET AL: "Combined expression of BUB1B, DLGAP5, and PINK1 as predictors of poor outcome in adrenocortical tumors: validation in a Brazilian cohort of adult and pediatric patients", EUROPEAN JOURNAL OF ENDOCRINOLOGY, vol. 166, 1 January 2012 (2012-01-01), pages 61 - 67, XP055772028
- ZHENG ET AL.: "Comprehensive Pan- Genomic Characterization of Adrenocortical Carcinoma", CANCER CELL, vol. 29, no. 5, 9 May 2016 (2016-05-09), pages 723 - 736, XP029533970, doi:10.1016/j.ccell.2016.04.002
- DE REYNIES ET AL.: "Gene Expression Profiling Reveals a New Classification of Adrenocortical Tumors and Identifies Molecular Predictors of Malignancy and Survival", JOURNAL OF CLINICAL ONCOLOGY, vol. 27, no. 7, 1 March 2009 (2009-03-01), pages 1108 - 1115, XP055618512
- LERARIO ET AL.: "Genetics and epigenetics of adrenocortical tumors", MOLECULAR AND CELLULAR ENDOCRINOLOGY, vol. 386, no. 1-2, 9 November 2013 (2013-11-09), pages 67 - 84, XP028662700, doi:10.1016/j.mce.2013.10.028
- BERRUTI ET AL.: "Adjuvant Therapy in Patients With Adrenocortical Carcinoma: A Position of an International Panel", JOURNAL OF CLINICAL ONCOLOGY, vol. 28, no. 23, 10 August 2010 (2010-08-10), pages e401 - e402, XP055618516

## Description

### CROSS REFERENCE TO RELATED APPLICATION

This application claims priority to and the benefit of U.S. Provisional Application No. 62/592,009, filed November 29, 2017 and U.S. Provisional Application No. 62/674,883, filed May 22, 2018, the contents of which are hereby incorporated by reference in their entireties.

### FIELD OF THE DISCLOSURE

The present disclosure relates to compositions, systems, and methods for characterizing cancer and determining a treatment course of action. In particular, the present disclosure relates to compositions, systems, and methods for utilizing gene expression and methylation profiles to stratify and treat adrenocortical carcinoma.

### BACKGROUND OF THE DISCLOSURE

Adrenocortical carcinoma (ACC) is a rare malignancy with an overall dismal prognosis. Treatment options for ACC are limited, and surgery is the only therapy that can provide long-term remission and cure. Despite surgery, a third of patients with early-stage disease develop metastases post-operatively and therefore require systemic treatment. For this reason, following margin-free surgical resection, adjuvant therapy with the adrenolytic compound mitotane is now part of the standard care for most ACC patients. Therapeutic serum levels of mitotane typically take several months of drug administration to achieve, and many patients inevitably recur during that window. Adjuvant cytotoxic chemotherapy is highly beneficial for these high-risk patients. Up to a third of patients do not recur following surgery and hence would not require adjuvant care. However, risk stratification in ACC is challenging.

The current approach to stratify risk of recurrence in patients with early-stage disease based on histological assessment of cell proliferation (either by the KI67 index or mitotic counts) has several limitations and does not reliably identify patients with high-risk ACC.

Lerario et al. (Molecular and cellular endocrinology, vol. 386, no. 1-2, 9 November 2013, pages 67-84) reviews the genetics and epigenetics of adrenocortical tumors. BUB1B and PINK1 are disclosed as expression markers, while G0S2 is described as being differentially methylated. Zheng et al.(Cancer Cell, vol. 29, no. 5, 9 May 2016, pages 723-736) describes a pan-genomic characterization of ACC.

Improved methods for identifying and treating high risk patients are needed.

### SUMMARY OF THE DISCLOSURE

The present disclosure relates to compositions, systems, and methods for characterizing cancer and determining a treatment course of action. In particular, the present disclosure relates to compositions, systems, and methods for utilizing gene expression and methylation profiles to stratify and treat adrenocortical carcinoma. The invention is defined by the claims. Any subject-matter falling outside the scope of the claims is provided for information purposes only.

In some embodiments, provided herein is a method for characterizing adrenocortical carcinoma (ACC), comprising or consisting of: a) contacting a sample from a subject diagnosed with ACC with reagents for determining the level of expression of at least one of BUB1 Mitotic Checkpoint Serine/Threonine Kinase B (BUB1B), PTEN Induced Putative Kinase 1 (PINK1), and G0/G1 Switch 2 (G0S2) and the methylation status of G0S2; and b) characterizing the ACC as molecular subgroup COC1, COC2, or COC3 based on the level of expression of at least one of BUB1B, PINK1, and G0S2 and methylation status of G0S2. In some embodiments, the characterizing comprises determining a *BUB1B-PINK1* expression score. In some embodiments, a *BUB1B-PINK1* expression score above a threshold level is indicative of COC1. In some embodiments, a *BUB1B-PINK1* expression score below a threshold level and less than a threshold level (e.g., 5%) G0S2 methylation is indicative of COC2. In some embodiments, a *BUB1B-PINK1* expression score below a threshold level and greater than a threshold level (e.g., 5%) G0S2 methylation is indicative of COC3. In some embodiments, the characterizing further comprises determining a prognosis (e.g., likelihood of metastasis). In some embodiments, COC3 classification is indicative of metastasis of the ACC and an aggressive cancer. In some embodiments, the method further comprises determining a treatment course of action based on the molecular subgroup. For example, in some embodiments, the treatment course of action comprises adjuvant cytotoxic chemotherapy (e.g., mitotane) in subjects with COC3 tumors. In some embodiments, the method further comprises administering the treatment.

Additional embodiments provide a method of treating ACC, comprising or consisting of: a) contacting a sample from a subject diagnosed with ACC with reagents for determining the level of expression of at least one of BUB1B, PINK1, and G0S2 and the methylation status of G0S2; b) characterizing the ACC as molecular subgroup COC1, COC2, or COC3 based on the level of expression of BUB1B, PINK1, and G0S2 and methylation status of G0S2; and c) administering adjuvant cytotoxic chemotherapy in subjects with COC3 tumors.

Yet other embodiments provide a method of assaying gene expression and methylation, comprising or consisting of: a) contacting a sample from a subject diagnosed with ACC with reagents for determining the level of expression of at least one of BUB1B, PINK1, and G0S2 and the methylation status of G0S2; and b) identifying the level of expression of BUB1B, PINK1, and G0S2 and methylation status of G0S2.

In some embodiments, the biological sample is, for example, a tissue sample, a biopsy sample, a blood sample, or a urine sample. In some embodiments, the reagents are, for example, a nucleic acid probe or probes that hybridizes to BUB1B, PINK1, and G0S2, one or more nucleic acid primers for the amplification or extension of BUB1B, PINK1, and G0S2, or one or more nucleic acid primers that bind specifically to methylated G0S2 nucleic acids or modified (e.g., bisulfite treated) G0S2 nucleic acids.

Still further embodiments provide a kit or system, comprising or consisting of: reagents for determining the level of expression of at least one of BUB1B, PINK1, and G0S2 and the methylation status of G0S2, wherein the reagents are, for example, a nucleic acid probe or probes that hybridizes to BUB1B, PINK1, and G0S2, one or more nucleic acid primers for the amplification or extension of BUB1B, PINK1, and G0S2, or one or more nucleic acid primers that bind specifically to methylated G0S2 nucleic acids modified (e.g., bisulfite treated) G0S2 nucleic acids. In some embodiments, the kit or system further comprises reagents for detection of methylated DNA (e.g., bisulfite), buffers, controls, and the like.

### DESCRIPTION OF THE DRAWINGS

FIG. 1. Molecular classification of ACC.
FIG. 2A-D. Characteristics of patient cohort.
FIG. 3. COC assignment flowchart.
FIG. 4A-G. Expression of *BUB1B* and *PINK1* and methylation of *G0S2* recapitulate COC1-3 from ACC-TCGA.

### DEFINITIONS

To facilitate an understanding of the present disclosure, a number of terms and phrases are defined below:
As used herein, the term "sensitivity" is defined as a statistical measure of performance of an assay (e.g., method, test), calculated by dividing the number of true positives by the sum of the true positives and the false negatives.

As used herein, the term "specificity" is defined as a statistical measure of performance of an assay (e.g., method, test), calculated by dividing the number of true negatives by the sum of true negatives and false positives.

As used herein, the term "informative" or "informativeness" refers to a quality of a marker or panel of markers, and specifically to the likelihood of finding a marker (or panel of markers) in a positive sample.

As used herein, the term "metastasis" is meant to refer to the process in which cancer cells originating in one organ or part of the body relocate to another part of the body and continue to replicate. Metastasized cells subsequently form tumors which may further metastasize. Metastasis thus refers to the spread of cancer from the part of the body where it originally occurs to other parts of the body. As used herein, the term "metastasized ACC cancer cells" is meant to refer to ACC cancer cells which have metastasized.

The term "neoplasm" as used herein refers to any new and abnormal growth of tissue. Thus, a neoplasm can be a premalignant neoplasm or a malignant neoplasm. The term "neoplasm-specific marker" refers to any biological material that can be used to indicate the presence of a neoplasm. Examples of biological materials include, without limitation, nucleic acids, polypeptides, carbohydrates, fatty acids, cellular components (e.g., cell membranes and mitochondria), and whole cells.

As used herein, the term "amplicon" refers to a nucleic acid generated using primer pairs. The amplicon is typically single-stranded DNA (e.g., the result of asymmetric amplification), however, it may be RNA or dsDNA.

The term "amplifying" or "amplification" in the context of nucleic acids refers to the production of multiple copies of a polynucleotide, or a portion of the polynucleotide, typically starting from a small amount of the polynucleotide (e.g., a single polynucleotide molecule), where the amplification products or amplicons are generally detectable. Amplification of polynucleotides encompasses a variety of chemical and enzymatic processes. The generation of multiple DNA copies from one or a few copies of a target or template DNA molecule during a polymerase chain reaction (PCR) or a ligase chain reaction (LCR; see, e.g., U.S. Patent No. 5,494,810) are forms of amplification. Additional types of amplification include, but are not limited to, allele-specific PCR (see, e.g., U.S. Patent No. 5,639,611), assembly PCR (see, e.g., U.S. Patent No. 5,965,408), helicase-dependent amplification (see, e.g., U.S. Patent No. 7,662,594), hot-start PCR (see, e.g., U.S. Patent Nos. 5,773,258 and 5,338,671), intersequence-specfic PCR, inverse PCR (see, e.g., Triglia, et al. (1988) Nucleic Acids Res., 16:8186), ligation-mediated PCR (see, e.g., Guilfoyle, R. et al., Nucleic Acids Research, 25:1854-1858 (1997); U.S. Patent No. 5,508,169), methylation-specific PCR (see, e.g., Herman, et al., (1996) PNAS 93(13) 9821-9826), miniprimer PCR, multiplex ligation-dependent probe amplification (see, e.g., Schouten, et al., (2002) Nucleic Acids Research 30(12): e5 7), multiplex PCR (see, e.g., Chamberlain, et al., (1988) Nucleic Acids Research 16(23) 11141-11156; Ballabio, et al., (1990) Human Genetics 84(6) 571-573; Hayden, et al., (2008) BMC Genetics 9:80), nested PCR, overlap-extension PCR (see, e.g., Higuchi, et al., (1988) Nucleic Acids Research 16(15) 7351-7367), real time PCR (see, e.g., Higuchi, etl al., (1992) Biotechnology 10:413-417; Higuchi, et al., (1993) Biotechnology 11:1026-1030), reverse transcription PCR (see, e.g., Bustin, S.A. (2000) J. Molecular Endocrinology 25:169-193), solid phase PCR, thermal asymmetric interlaced PCR, and Touchdown PCR (see, e.g., Don, et al., Nucleic Acids Research (1991) 19(14) 4008; Roux, K. (1994) Biotechniques 16(5) 812-814; Hecker, et al., (1996) Biotechniques 20(3) 478-485). Polynucleotide amplification also can be accomplished using digital PCR (see, e.g., Kalinina, et al., Nucleic Acids Research. 25; 1999-2004, (1997); Vogelstein and Kinzler, Proc Natl Acad Sci USA. 96; 9236-41, (1999); International Patent Publication No. WO05023091A2; US Patent Application Publication No. 20070202525).

As used herein, the terms "complementary" or "complementarity" are used in reference to polynucleotides (*i.e.,* a sequence of nucleotides) related by the base-pairing rules. For example, the sequence "5'-A-G-T-3'," is complementary to the sequence "3'-T-C-A-5'." Complementarity may be "partial," in which only some of the nucleic acids' bases are matched according to the base pairing rules. Or, there may be "complete" or "total" complementarity between the nucleic acids. The degree of complementarity between nucleic acid strands has significant effects on the efficiency and strength of hybridization between nucleic acid strands. This is of particular importance in amplification reactions, as well as detection methods that depend upon binding between nucleic acids.

As used herein, the term "primer" refers to an oligonucleotide, whether occurring naturally as in a purified restriction digest or produced synthetically, that is capable of acting as a point of initiation of synthesis when placed under conditions in which synthesis of a primer extension product that is complementary to a nucleic acid strand is induced (*e.g*., in the presence of nucleotides and an inducing agent such as a biocatalyst (*e.g.*, a DNA polymerase or the like) and at a suitable temperature and pH). The primer is typically single stranded for maximum efficiency in amplification, but may altematively be double stranded. If double stranded, the primer is generally first treated to separate its strands before being used to prepare extension products. In some embodiments, the primer is an oligodeoxyribonucleotide. The primer is sufficiently long to prime the synthesis of extension products in the presence of the inducing agent. The exact lengths of the primers will depend on many factors, including temperature, source of primer and the use of the method. In certain embodiments, the primer is a capture primer.

As used herein, the term "nucleic acid molecule" refers to any nucleic acid containing molecule, including but not limited to, DNA or RNA. The term encompasses sequences that include any of the known base analogs of DNA and RNA including, but not limited to, 4 acetylcytosine, 8-hydroxy-N6-methyladenosine, aziridinylcytosine, pseudoisocytosine, 5-(carboxyhydroxyl-methyl) uracil, 5-fluorouracil, 5-bromouracil, 5-carboxymethylaminomethyl-2-thiouracil, 5-carboxymethyl-aminomethyluracil, dihydrouracil, inosine, N6-isopentenyladenine, 1-methyladenine, 1-methylpseudo-uracil, 1-methylguanine, 1-methylinosine, 2,2-dimethyl-guanine, 2-methyladenine, 2-methylguanine, 3-methyl-cytosine, 5-methylcytosine, N6-methyladenine, 7-methylguanine, 5-methylaminomethyluracil, 5-methoxy-amino-methyl-2-thiouracil, beta-D-mannosylqueosine, 5'-methoxycarbonylmethyluracil, 5-methoxyuracil, 2-methylthio-N- isopentenyladenine, uracil-5-oxyacetic acid methylester, uracil-5-oxyacetic acid, oxybutoxosine, pseudouracil, queosine, 2-thiocytosine, 5-methyl-2-thiouracil, 2-thiouracil, 4-thiouracil, 5-methyluracil, N-uracil-5-oxyacetic acid methylester, uracil-5-oxyacetic acid, pseudouracil, queosine, 2-thiocytosine, and 2,6-diaminopurine.

As used herein, the term "nucleobase" is synonymous with other terms in use in the art including "nucleotide," "deoxynucleotide," "nucleotide residue," "deoxynucleotide residue," "nucleotide triphosphate (NTP)," or deoxynucleotide triphosphate (dNTP).

An "oligonucleotide" refers to a nucleic acid that includes at least two nucleic acid monomer units (e.g., nucleotides), typically more than three monomer units, and more typically greater than ten monomer units. The exact size of an oligonucleotide generally depends on various factors, including the ultimate function or use of the oligonucleotide. To further illustrate, oligonucleotides are typically less than 200 residues long (e.g., between 15 and 100), however, as used herein, the term is also intended to encompass longer polynucleotide chains. Oligonucleotides are often referred to by their length. For example a 24 residue oligonucleotide is referred to as a "24-mer". Typically, the nucleoside monomers are linked by phosphodiester bonds or analogs thereof, including phosphorothioate, phosphorodithioate, phosphoroselenoate, phosphorodiselenoate, phosphoroanilothioate, phosphoranilidate, phosphoramidate, and the like, including associated counterions, e.g., H⁺, NH₄⁺, Na⁺, and the like, if such counterions are present. Further, oligonucleotides are typically single-stranded. Oligonucleotides are optionally prepared by any suitable method, including, but not limited to, isolation of an existing or natural sequence, DNA replication or amplification, reverse transcription, cloning and restriction digestion of appropriate sequences, or direct chemical synthesis by a method such as the phosphotriester method of Narang et al. (1979) Meth Enzymol. 68: 90-99; the phosphodiester method of Brown et al. (1979) Meth Enzymol. 68: 109-151; the diethylphosphoramidite method of Beaucage et al. (1981) Tetrahedron Lett. 22: 1859-1862; the triester method of Matteucci et al. (1981) J Am Chem Soc. 103:3185-3191; automated synthesis methods; or the solid support method of U.S. Pat. No. 4,458,066, entitled "PROCESS FOR PREPARING POLYNUCLEOTIDES," issued Jul. 3, 1984 to Caruthers et al., or other methods known to those skilled in the art.

A "sequence" of a biopolymer refers to the order and identity of monomer units (e.g., nucleotides, etc.) in the biopolymer. The sequence (e.g., base sequence) of a nucleic acid is typically read in the 5' to 3' direction.

As used herein, "methylation" refers to cytosine methylation at positions C5 or N4 of cytosine, the N6 position of adenine, or other types of nucleic acid methylation. *In vitro* amplified DNA is unmethylated because *in vitro* DNA amplification methods do not retain the methylation pattern of the amplification template. However, "unmethylated DNA" or "methylated DNA" can also refer to amplified DNA whose original template was unmethylated or methylated, respectively.

"Methylation status" refers to the presence, absence, and/or quantity of methylation at a particular nucleotide or nucleotides within a portion of DNA. The methylation status of a particular DNA sequence (e.g., a gene marker or DNA region as described herein) can indicate the methylation state of every base in the sequence or can indicate the methylation state of a subset of the base pairs (e.g., of one or more cytosines) within the sequence, or can indicate information regarding regional methylation density within the sequence without providing precise information of where in the sequence the methylation occurs. The methylation status can optionally be represented or indicated by a "methylation value." A methylation value can be generated, for example, by quantifying the amount of intact DNA present following restriction digestion with a methylation dependent restriction enzyme or by comparing amplification profiles after bisulfite reaction or by comparing sequences of bisulfite-treated and untreated DNA. Accordingly, a value, e.g., a methylation value, represents the methylation status and can thus be used as a quantitative indicator of methylation status across multiple copies of a locus. This is of particular use when it is desirable to compare the methylation status of a sequence in a sample to a threshold or reference value.

As used herein, the term "subject" refers to any animal (e.g., a mammal), including, but not limited to, humans, non-human primates, rodents, and the like, which is to be the recipient of a particular treatment. Typically, the terms "subject" and "patient" are used interchangeably herein in reference to a human subject.

As used herein, the term "non-human animals" refers to all non-human animals including, but are not limited to, vertebrates such as rodents, non-human primates, ovines, bovines, ruminants, lagomorphs, porcines, caprines, equines, canines, felines, aves, etc.

The term "gene" refers to a nucleic acid (*e.g.,* DNA) sequence that comprises coding sequences necessary for the production of a polypeptide, RNA (*e.g*., including but not limited to, mRNA, tRNA and rRNA) or precursor. The polypeptide, RNA, or precursor can be encoded by a full length coding sequence or by any portion of the coding sequence so long as the desired activity or functional properties (*e*.*g*., enzymatic activity, ligand binding, signal transduction, etc.) of the full-length or fragment are retained. The term also encompasses the coding region of a structural gene and the including sequences located adjacent to the coding region on both the 5' and 3' ends for a distance of about 1 kb on either end such that the gene corresponds to the length of the full-length mRNA. The sequences that are located 5' of the coding region and which are present on the mRNA are referred to as 5' untranslated sequences. The sequences that are located 3' or downstream of the coding region and that are present on the mRNA are referred to as 3' untranslated sequences. The term "gene" encompasses both cDNA and genomic forms of a gene. A genomic form or clone of a gene contains the coding region interrupted with non-coding sequences termed "introns" or "intervening regions" or "intervening sequences". Introns are segments of a gene that are transcribed into nuclear RNA (hnRNA); introns may contain regulatory elements such as enhancers. Introns are removed or "spliced out" from the nuclear or primary transcript; introns therefore are absent in the messenger RNA (mRNA) processed transcript. The mRNA functions during translation to specify the sequence or order of amino acids in a nascent polypeptide.

The term "locus" as used herein refers to a nucleic acid sequence on a chromosome or on a linkage map and includes the coding sequence as well as 5' and 3' sequences involved in regulation of the gene.

### DETAILED DESCRIPTION OF THE DISCLOSURE

The present disclosure relates to compositions, systems, and methods for characterizing cancer and determining a treatment course of action. In particular, the present disclosure relates to compositions, systems, and methods for utilizing gene expression and methylation profiles to stratify and treat adrenocortical carcinoma.

Recently, it was revealed that ACC is comprised of three molecular subtypes, "COC1," "COC2," and "COC3." While COC1 and COC2 tumors exhibit more favorable and intermediate prognoses, respectively, COC3 tumors present with uniformly dismal outcomes (median event-free survival of 8 months). COC3 tumors are also distinguished by many targetable molecular alterations. These tumors often bear genetic alterations in cell-cycle regulators, several chromosomal breakpoints, and widespread CpG island hypermethylation ("CIMP-high"). Therefore, stratification of ACC based on the TCGA molecular classes is a reliable method of risk assessment with clinical value. Specifically, reliably distinguishing COC3 tumors from other ACC subtypes facilitates the development and implementation of new adjuvant strategies that benefit these high-risk patients and prevent the application of chemotherapy in lower risk patients. Importantly, an approach that uses few biomarkers to recapitulate the findings of the TCGA and that allows the stratification of patients in a time frame compatible with clinical decision making is useful.

Accordingly, provided herein is a method of classifying ACC comprising evaluating the expression of few key genes and DNA methylation of a single locus to stratify ACC samples according to the molecular categories defined by the ACC-TCGA.

### I. Identification of ACC classification

As described herein, in some embodiments, the expression or level of one or more ACC markers (e.g., BUB1B, PINK1, or G0S2) and methylation status of G0S2. In some embodiments, an expression and/or methylation score is used to characterize ACC. For example, in some embodiments, a *BUB1B-PINK1* expression score above a threshold level is indicative of COC1. In some embodiments, a *BUB1B PINK1* expression score below a threshold level and less than a threshold level (e.g., 5%) G0S2 methylation is indicative of COC2. In some embodiments, a *BUB1B-PINK1* expression score below a threshold level and greater than a threshold level (e.g., 5%) G0S2 methylation is indicative of COC3. (See e.g., Examples 1 and 2 below).

Exemplary detection and scoring methods are described below.

### A. DNA and RNA Detection

In some embodiments, RNA is detection by Northern blot analysis. Northern blot analysis involves the separation of RNA and hybridization of a complementary labeled probe. In some embodiments, RNA (or corresponding cDNA) is detected by hybridization to a oligonucleotide probe). A variety of hybridization assays using a variety of technologies for hybridization and detection are available. For example, in some embodiments, TaqMan assay (PE Biosystems, Foster City, CA; *See e.g.,* U.S. Patent Nos. 5,962,233 and 5,538, 848) is utilized. The assay is performed during a PCR reaction. The TaqMan assay exploits the 5'-3' exonuclease activity of the AMPLITAQ GOLD DNA polymerase. A probe consisting of an oligonucleotide with a 5'-reporter dye (*e.g.,* a fluorescent dye) and a 3'-quencher dye is included in the PCR reaction. During PCR, if the probe is bound to its target, the 5'-3' nucleolytic activity of the AMPLITAQ GOLD polymerase cleaves the probe between the reporter and the quencher dye. The separation of the reporter dye from the quencher dye results in an increase of fluorescence. The signal accumulates with each cycle of PCR and can be monitored with a fluorimeter.

In some embodiments, microarrays including, but not limited to: DNA microarrays (*e.g*., cDNA microarrays and oligonucleotide microarrays); protein microarrays; tissue microarrays; transfection or cell microarrays; chemical compound microarrays; and, antibody microarrays are utilized for measuring cancer marker mRNA levels. A DNA microarray, commonly known as gene chip, DNA chip, or biochip, is a collection of microscopic DNA spots attached to a solid surface (e.g., glass, plastic or silicon chip) forming an array for the purpose of expression profiling or monitoring expression levels for thousands of genes simultaneously. The affixed DNA segments are known as probes, thousands of which can be used in a single DNA microarray. Microarrays can be used to identify disease genes by comparing gene expression in disease and normal cells. Microarrays can be fabricated using a variety of technologies, including but not limited to: printing with fine-pointed pins onto glass slides; photolithography using pre-made masks; photolithography using dynamic micromirror devices; ink-jet printing; or, electrochemistry on microelectrode arrays.

In yet other embodiments, reverse-transcriptase PCR (RT-PCR) is used to detect the expression of RNA. In RT-PCR, RNA is enzymatically converted to complementary DNA or "cDNA" using a reverse transcriptase enzyme. The cDNA is then used as a template for a PCR reaction. PCR products can be detected by any suitable method, including but not limited to, gel electrophoresis and staining with a DNA specific stain or hybridization to a labeled probe. In some embodiments, the quantitative reverse transcriptase PCR with standardized mixtures of competitive templates method described in U.S. Patents 5,639,606, 5,643,765, and 5,876,978 is utilized. In some embodiments, the cancer markers are detected by hybridization with a detectably labeled probe and measurement of the resulting hybrids. Illustrative non-limiting examples of detection methods are described below.

One illustrative detection method, the Hybridization Protection Assay (HPA) involves hybridizing a chemiluminescent oligonucleotide probe (*e.g.,* an acridinium ester-labeled (AE) probe) to the target sequence, selectively hydrolyzing the chemiluminescent label present on unhybridized probe, and measuring the chemiluminescence produced from the remaining probe in a luminometer. *See, e.g.,* U.S. Pat. No. 5,283,174; Nelson et al., Nonisotopic Probing, Blotting, and Sequencing, ch. 17 (Larry J. Kricka ed., 2d ed. 1995).

The interaction between two molecules can also be detected, *e.g*., using fluorescence energy transfer (FRET) (see, for example, Lakowicz et al., U.S. Pat. No. 5,631,169; Stavrianopoulos et al., U.S. Pat. No. 4,968,103). A fluorophore label is selected such that a first donor molecule's emitted fluorescent energy will be absorbed by a fluorescent label on a second, 'acceptor' molecule, which in turn is able to fluoresce due to the absorbed energy.

Alternately, the 'donor' protein molecule may simply utilize the natural fluorescent energy of tryptophan residues. Labels are chosen that emit different wavelengths of light, such that the 'acceptor' molecule label may be differentiated from that of the 'donor'. Since the efficiency of energy transfer between the labels is related to the distance separating the molecules, the spatial relationship between the molecules can be assessed. In a situation in which binding occurs between the molecules, the fluorescent emission of the 'acceptor' molecule label should be maximal. A FRET binding event can be conveniently measured through fluorometric detection means.

Another example of a detection probe having self-complementarity is a "molecular beacon." Molecular beacons include nucleic acid molecules having a target complementary sequence, an affinity pair (or nucleic acid arms) holding the probe in a closed conformation in the absence of a target sequence present in an amplification reaction, and a label pair that interacts when the probe is in a closed conformation. Hybridization of the target sequence and the target complementary sequence separates the members of the affinity pair, thereby shifting the probe to an open conformation. The shift to the open conformation is detectable due to reduced interaction of the label pair, which may be, for example, a fluorophore and a quencher (e.g., DABCYL and EDANS). Molecular beacons are disclosed, for example, in U.S. Pat. Nos. 5,925,517 and 6,150,097. By way of non-limiting example, probe binding pairs having interacting labels, such as those disclosed in U.S. Pat. No. 5,928,862 might be adapted for use in meothd of embodiments of the present disclsoure. Probe systems used to detect single nucleotide polymorphisms (SNPs) might also be utilized in the present invention. Additional detection systems include "molecular switches," as disclosed in U.S. Publ. No. 20050042638. Other probes, such as those comprising intercalating dyes and/or fluorochromes, are also useful for detection of amplification products methods of embodiments of the present disclosure. *See, e.g.,* U.S. Pat. No. 5,814,447.

In some embodiments, nucleic acid sequencing methods are utilized for detection. In some embodiments, the sequencing is Second Generation (a.k.a. Next Generation or Next-Gen), Third Generation (a.k.a. Next-Next-Gen), or Fourth Generation (a.k.a. N3-Gen) sequencing technology including, but not limited to, pyrosequencing, sequencing-by-ligation, single molecule sequencing, sequence-by-synthesis (SBS), semiconductor sequencing, massive parallel clonal, massive parallel single molecule SBS, massive parallel single molecule real-time, massive parallel single molecule real-time nanopore technology, etc. Morozova and Marra provide a review of some such technologies in Genomics, 92: 255 (2008). Those of ordinary skill in the art will recognize that because RNA is less stable in the cell and more prone to nuclease attack experimentally RNA is usually reverse transcribed to DNA before sequencing.

DNA sequencing techniques include fluorescence-based sequencing methodologies (See, e.g., Birren et al., Genome Analysis: Analyzing DNA, 1, Cold Spring Harbor, N.Y. In some embodiments, the sequencing is automated sequencing. In some embodiments, the sequenceing is parallel sequencing of partitioned amplicons (PCT Publication No: WO2006084132 to Kevin McKernan et al.).

In some embodiments, the sequencing is DNA sequencing by parallel oligonucleotide extension (See, e.g., U.S. Pat. No. 5,750,341 to Macevicz et al., and U.S. Pat. No. 6,306,597 to Macevicz et al. Additional examples of sequencing techniques include the Church polony technology (Mitra et al., 2003, Analytical Biochemistry 320, 55-65; Shendure et al., 2005 Science 309, 1728-1732; U.S. Pat. No. 6,432,360, U.S. Pat. No. 6,485,944, U.S. Pat. No. 6,511,803), the 454 picotiter pyrosequencing technology (Margulies et al., 2005 Nature 437, 376-380; US 20050130173), the Solexa single base addition technology (Bennett et al., 2005, Pharmacogenomics, 6, 373-382; U.S. Pat. No. 6,787,308; U.S. Pat. No. 6,833,246), the Lynx massively parallel signature sequencing technology (Brenner et al. (2000). Nat. Biotechnol. 18:630-634; U.S. Pat. No. 5,695,934; U.S. Pat. No. 5,714,33 0), and the Adessi PCR colony technology (Adessi et al. (2000). Nucleic Acid Res. 28, E87; WO 00018957).

Next-generation sequencing (NGS) methods share the common feature of massively parallel, high-throughput strategies, with the goal of lower costs in comparison to older sequencing methods (see, e.g., Voelkerding et al., Clinical Chem., 55: 641-658, 2009; MacLean et al., Nature Rev. Microbiol., 7: 287-296). NGS methods can be broadly divided into those that typically use template amplification and those that do not. Amplification-requiring methods include pyrosequencing commercialized by Roche as the 454 technology platforms (e.g., GS 20 and GS FLX), Life Technologies/Ion Torrent, the Solexa platform commercialized by Illumina, GnuBio, and the Supported Oligonucleotide Ligation and Detection (SOLiD) platform commercialized by Applied Biosystems. Non-amplification approaches, also known as single-molecule sequencing, are exemplified by the HeliScope platform commercialized by Helicos BioSciences, and emerging platforms commercialized by VisiGen, Oxford Nanopore Technologies Ltd., and Pacific Biosciences, respectively.

### B. Methylation

In mammals, methylation occurs only at cytosine residues and more specifically only on a cytosine residue that is adjacent to a guanine residue (that is, at the sequence CG, often denoted "CpG"). Detecting and mapping sites of DNA methylation are essential steps for understanding epigenetic gene regulation and providing diagnostic tools for identifying cancers and other disease states associated with errors in gene regulation.

Mapping the state of DNA methylation at particular sites is currently accomplished by the bisulfite method described by Frommer, et al. for the detection of 5-methylcytosines in DNA (Proc. Natl. Acad. Sci. USA 89: 1827-31 (1992) or variations thereof. The bisulfite method of mapping 5-methylcytosines is based on the observation that cytosine, but not 5-methylcytosine, reacts with hydrogen sulfite ion (also known as bisulfite). The reaction is usually performed according to the following steps: first, cytosine reacts with hydrogen sulfite to form a sulfonated cytosine. Next, spontaneous deamination of the sulfonated reaction intermediate results in a sulfonated uracil. Finally, the sulfonated uricil is desulfonated under alkaline conditions to form uracil. Detection is possible because uracil forms base pairs with adenine (thus behaving like thymine), whereas 5-methylcytosine base pairs with guanine (thus behaving like cytosine). This makes the discrimination of methylated cytosines from non-methylated cytosines possible by, e.g., bisulfite genomic sequencing (Grigg G, & Clark S, Bioessays (1994) 16: 431-36; Grigg G, DNA Seq. (1996) 6: 189-98) or methylation-specific PCR (MSP) as is disclosed, e.g., in U.S. Patent No. 5,786,146.

A gene's methylation state is often expressed as the fraction or percentage of individual strands of DNA that are methylated at a particular site (e.g., at a single nucleotide or at a longer sequence of interest, e.g., up to a ~100-bp subsequence of a DNA) relative to the total population of DNA in the sample comprising that particular site. Traditionally, the amount of unmethylated (e.g., native) gene is determined by PCR using calibrators. Then, a known amount of DNA is bisulphite treated and the resulting methylation-specific sequence is determined using either a real-time PCR or an equivalent exponential amplification.

For example, conventional methods generally comprise generating a standard curve for the unmethylated target by using external standards. The standard curve is constructed from at least two points and relates the real-time Ct value for unmethylated DNA to known quantitative standards. Then, a second standard curve for the methylated target is constructed from at least two points and external standards. This second standard curve relates the Ct for methylated DNA to known quantitative standards. Next, the test sample Ct values are determined for the methylated and unmethylated populations and the genomic equivalents of DNA are calculated from the standard curves produced by the first two steps. The percentage of methylation at the site of interest is calculated from the amount of methylated DNAs relative to the total amount of DNAs in the population, e.g., (number of methylated DNAs) / (the number of methylated DNAs + number of unmethylated DNAs) × 100.

The present disclosure is not restricted in the method by which a gene's methylation state is measured. For example, in some embodiments the methylation state is measured by a genome scanning methods. For example, one method involves restriction landmark genomic scanning (Kawai et al., Mol. Cell. Biol. 14:7421-7427, 1994) and another example involves methylation-sensitive arbitrarily primed PCR (Gonzalgo et al., Cancer Res. 57:594-599, 1997). In some embodiments, changes in methylation patterns at specific CpG sites are monitored by digestion of genomic DNA with methylation-sensitive restriction enzymes followed by Southern analysis of the regions of interest (digestion-Southern method). In some embodiments, analyzing changes in methylation patterns involves a PCR-based process that involves digestion of genomic DNA with methylation-sensitive restriction enzymes prior to PCR amplification (Singer-Sam et al., Nucl. Acids Res. 18:687, 1990). In addition, other techniques have been reported that utilize bisulfite treatment of DNA as a starting point for methylation analysis. These include methylation-specific PCR (MSP) (Herman et al. Proc. Natl. Acad. Sci. USA 93:9821-9826, 1992) and restriction enzyme digestion of PCR products amplified from bisulfite-converted DNA (Sadri and Hornsby, Nucl. Acids Res. 24:5058-5059, 1996; and Xiong and Laird, Nucl. Acids Res. 25:2532-2534, 1997). PCR techniques have been developed for detection of gene mutations (Kuppuswamy et al., Proc. Natl. Acad. Sci. 55 USA 88:1143-1147, 1991) and quantification of allelic-specific expression (Szabo and Mann, Genes Dev. 9:3097-3108, 1995; and Singer-Sam et al., PCR Methods Appl. 1:160-163, 1992). Such techniques use internal primers, which anneal to a PCR-generated template and terminate immediately 5' of the single nucleotide to be assayed. Methods using a "quantitative Ms-SNuPE assay"as described in U.S. Pat. No. 7,037,650 are used in some embodiments.

### II. Prognosis and treatment of ACC

Further embodiments provide methods of characterizing ACC (e.g., to provide a prognosis such as tumor aggressiveness or likelihood of metastasis or determine a treatment course of action). For example, in some embodiments, COC3 classification is indicative of metastasis of the ACC and an aggressive cancer. In some embodiments, subjects with COC3 tumors are administered adjuvant cytotoxic chemotherapy (e.g., mitotane) and subjects with COC1 or COC2 tumors are treated with surgery alone. In some embodiments, the COC classification determination is repeated (e.g., during treatment or after surgery).

In some embodiments, a computer-based analysis program is used to translate the raw data generated by the detection assay (e.g., the expression level a given marker or markers) into data of predictive value for a clinician. The clinician can access the predictive data using any suitable means. Thus, in some preferred embodiments, the present disclosure provides the further benefit that the clinician, who is not likely to be trained in genetics or molecular biology, need not understand the raw data. The data is presented directly to the clinician in its most useful form. The clinician is then able to immediately utilize the information in order to optimize the care of the subject.

The present disclosure contemplates any method capable of receiving, processing, and transmitting the information to and from laboratories conducting the assays, information provides, medical personal, and subjects. For example, in some embodiments of the present disclosure, a sample (*e.*g., a biopsy or a blood or urine sample) is obtained from a subject and submitted to a profiling service (*e.g*., clinical lab at a medical facility, genomic profiling business, etc.), located in any part of the world (*e.g.*, in a country different than the country where the subject resides or where the information is ultimately used) to generate raw data. Where the sample comprises a tissue or other biological sample, the subject may visit a medical center to have the sample obtained and sent to the profiling center, or subjects may collect the sample themselves (*e.g*., a urine sample) and directly send it to a profiling center. Where the sample comprises previously determined biological information, the information may be directly sent to the profiling service by the subject (*e.g.*, an information card containing the information may be scanned by a computer and the data transmitted to a computer of the profiling center using an electronic communication systems). Once received by the profiling service, the sample is processed and a profile is produced (*i.e.,* expression data), specific for the diagnostic or prognostic information desired for the subject.

The profile data is then prepared in a format suitable for interpretation by a treating clinician. For example, rather than providing raw expression data, the prepared format may represent a diagnosis or risk assessment for the subject, along with recommendations for particular treatment options. The data may be displayed to the clinician by any suitable method. For example, in some embodiments, the profiling service generates a report that can be printed for the clinician (*e.g.,* at the point of care) or displayed to the clinician on a computer monitor.

In some embodiments, the information is first analyzed at the point of care or at a regional facility. The raw data is then sent to a central processing facility for further analysis and/or to convert the raw data to information useful for a clinician or patient. The central processing facility provides the advantage of privacy (all data is stored in a central facility with uniform security protocols), speed, and uniformity of data analysis. The central processing facility can then control the fate of the data following treatment of the subject. For example, using an electronic communication system, the central facility can provide data to the clinician, the subject, or researchers.

In some embodiments, the subject is able to directly access the data using the electronic communication system. The subject may chose further intervention or counseling based on the results. In some embodiments, the data is used for research use. For example, the data may be used to further optimize the inclusion or elimination of markers as useful indicators of a particular condition or stage of disease or as a companion diagnostic to determine a treatment course of action.

### III. Compositions & Kits

Compositions for use in the methods described herein include, but are not limited to, kits comprising one or more reagents for determining the level of expression of BUB1B, PINK1, and G0S2 and the methylation status of G0S2 as described above. In some embodiments, the reagents are, for example, a nucleic acid probe or probes that hybridizes to BUB1B, PINK1, and G0S2, one or more nucleic acid primers for the amplification or extension of BUB1B, PINK1, and G0S2, or one or more nucleic acid primers that bind specifically to methylated G0S2 nucleic acids.

The probes may also be provided in the form of an array. In preferred embodiments, the kits contain all of the components necessary to perform a detection assay, including all controls, directions for performing assays, and any necessary software for analysis and presentation of results.

### EXPERIMENTAL

The following examples are provided in order to demonstrate and further illustrate certain preferred embodiments and aspects of the present disclosure and are not to be construed as limiting the scope thereof.

### Example 1

Using a logistic regression model, it was determined that the expression level of *G0S2* can reliably distinguish COC3 from COC2 as *G0S2* is silenced in COC3. The promoter region of *G0S2* features a large CpG island that is completely methylated exclusively in CIMP-high tumors, and promoter methylation is strongly correlated with *G0S2* silencing. To expand on this observation, targeted bisulfite sequencing of the *G0S2* promoter in a panel of samples, including normal tissues (lung, kidney, liver, ovary, adrenal cortex), adrenocortical adenomas, ACCs, and the human ACC-derived NCI-H295R cell line was performed. NCIH295R was included in this panel because this cell line has several molecular aberrations consistent with a TCGA classification of COC3. *G0S2* methylation was observed in a subset of ACCs and the NCI-H295R cell line. Samples exhibited an "all or none" pattern of *G0S2* methylation; the entire locus was either completely methylated or unmethylated, also consistent with the TCGA. Additionally, the transcriptome and methylome of the NCI-H295R was characterized by RNA-Seq and the Illumina 850k EPIC array, and it was determined that this cell line has no detectable expression of *G0S2* and has methylation of all probes spanning the *G0S2* CpG island. Finally, the EpiTect Methyl II PCR Assay (Qiagen) was used as a rapid, bench-top assay to assess *G0S2* methylation status in the NCI-H295R cell line, and it was confirmed that the locus is >99% methylated.

A study is performed with ~100 ACC fresh-frozen samples with well-annotated clinical follow-up. The expression levels of *BUB1B, PINK1,* and *G0S2* are measured by TaqMan^{®} assays and the methylation status of the *G0S2* promoter region by the EpiTect Methyl II PCR Assay (Qiagen). As an additional validation, the methylation status of *G0S2* is confirmed by bisulfite sequencing in a subset of samples. These biomarkers are used to stratify patients according to the schema in **Table 1.** The log-rank test is used to compare the survival times of these different subgroups. Finally, a Cox proportional hazards model is used to calculate the hazard ratios and to perform multivariate analysis.

**Table 1. TCGA molecular classification.**

| **TCGA Cluster** | **Prognosis** | **ΔC_{T} *BUB1B* - ΔC_{T} *PINK1*** | **ΔC_{T} *G052*** | ***G0S2* methylation** |
|---|---|---|---|---|
| COC1 | Good (EFS^{*} > 10 yrs) | High^{↔} | Present ΔC_{T}≤ 10) | Low (<10%) |
| COC2 | Itermediate (EFS = 4 yrs) | Low^{↔} | Present ΔC_{T}≤ 10) | Low (<10%) |
| COC3 | Dismal (EFS < 1 yr) | Low^{↔} | Absent (ΔC_{T}>10) | High (>90%) |

| | | | | |
|---|---|---|---|---|
| *EFS = Median event-free survival: **Cutoffs will be rederived experimentally | | | | |

### Example 2

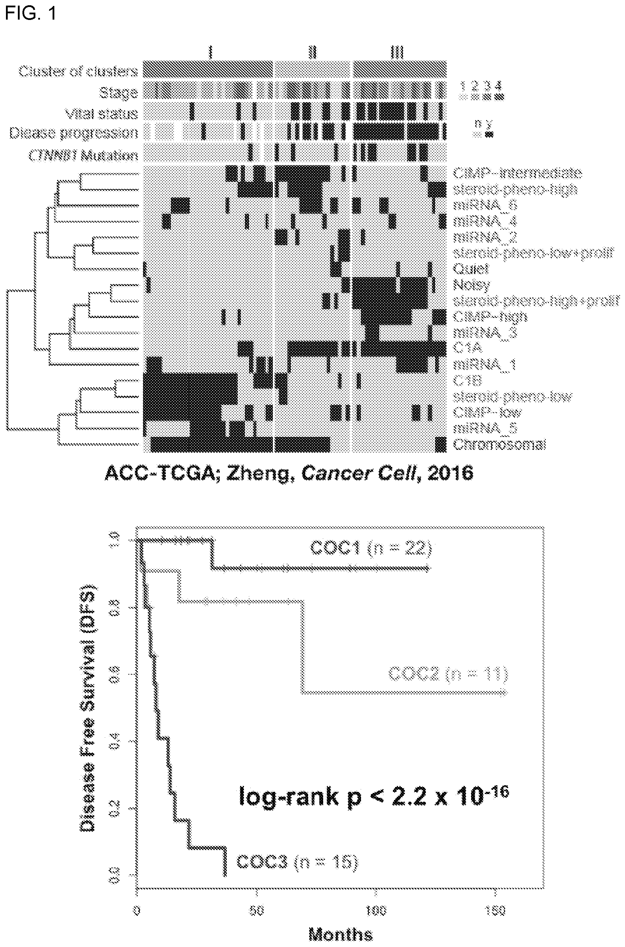

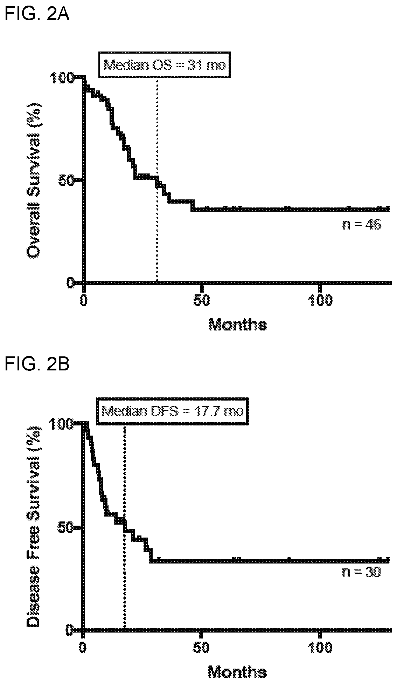

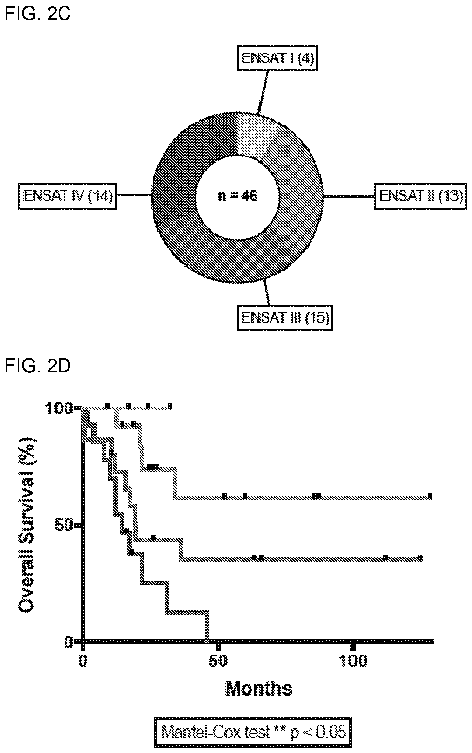

This example describes the evaluating the expression of BUB1B and PINK1 and methylation of G0S2 to stratify ACC samples according to the COC assignments of the ACC-TCGA (FIG. 1). Multiplatform analysis of ACC-TCGA demonstrates that ACC is comprised of 3 molecular subtypes with distinct disease-free survival. Figure 2 shows the patient cohort used in studies. The cohort includes primary tumors from 46 adult ACC patients. Overall survival and disease free survival are depicted in A and B, respectively. Clinical staging was performed at diagnosis following ENSAT criteria, depicted in panel C. Overall survival according to ENSAT stage at diagnosis shown in D.

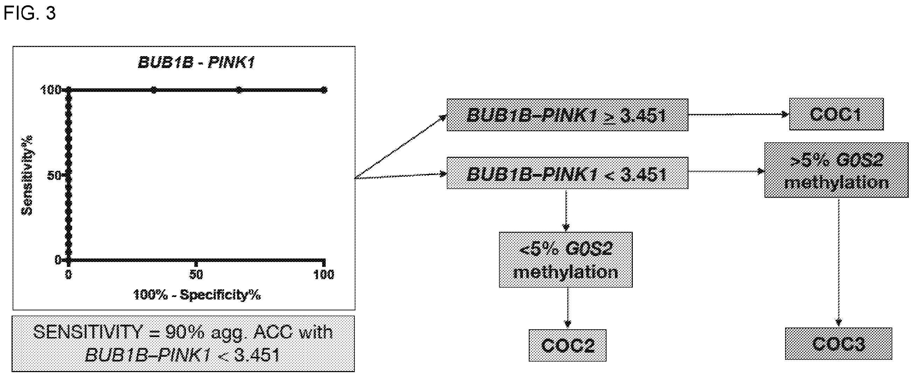

Figure 3 shows an assignment of COC catagories. Total mRNA and gDNA were harvested from 27 of 46 frozen primary tumor samples. Expression of *BUB1B, PINK1, G0S2,* and housekeeping gene *GUSB* were evaluated by TaqMan assays. The methylation of *G0S2* was evaluated by methylation-sensitive qPCR. The ROC-curve method was used to determine the cutoff for *Δ_{CT}*(*BUB1B-PINK1*) according to clinical outcomes. A "COC1" *BUB1B-PINK1* score was assigned to patients disease free with > 2 years of clinical observation after R0 resection and no evidence of metastasis at any time point.

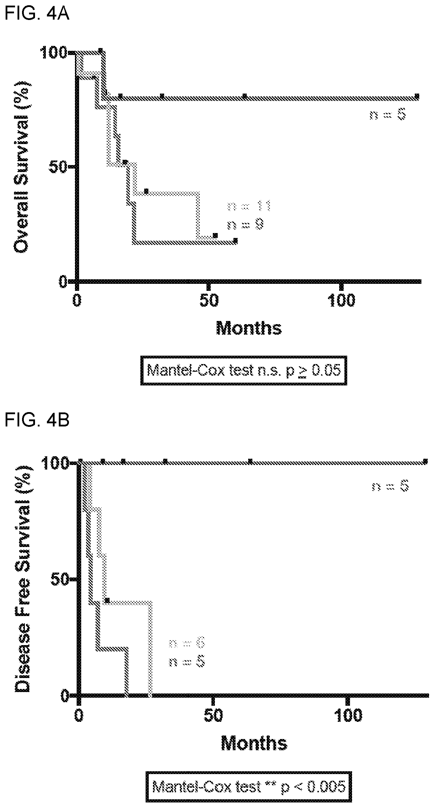

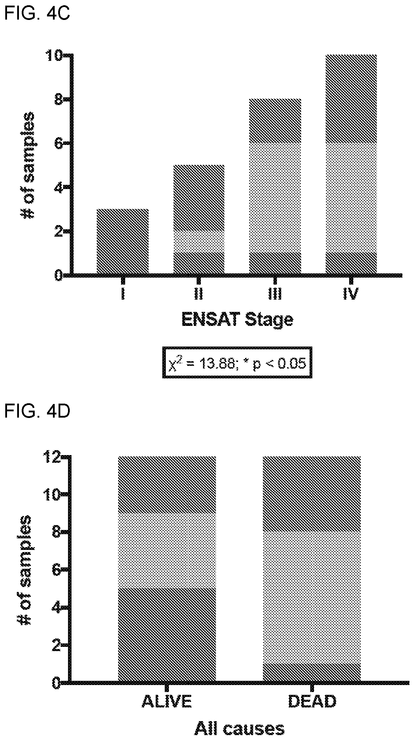

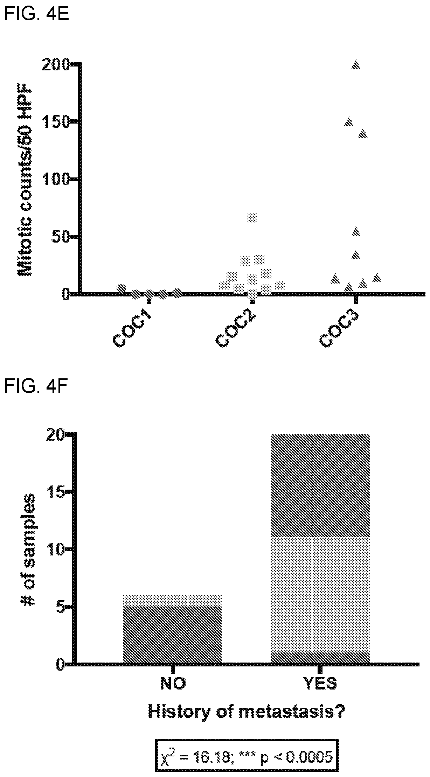

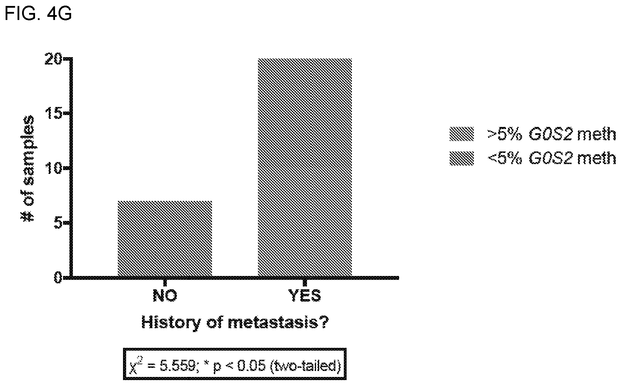

Figure 4 shows that expression of BUB1B and PINK1 and methylation of G0S2 recapitulate COC1-3 from ACC-TCGA in an independent cohort. BUB1B-PINK1 and G0S2 methylation stratify the FMUSP cohort into 3 distinct clinical subgroups (B). The COC3-designated tumors are also associated with clinical and histological markers of aggressiveness (C, E). Finally, in the cohort, COC3 assignment and G0S2 methylation are invariably predictive of metastasis (F, G).

In some embodiments, ΔC_{T} *G0S2* and the methylation status of the *G0S2* CpG island is used to assign patients to COC3 (> *5% G0S2* methylation +/- ΔC_{T} *G0S2 >* 5). In some embodiments, in the event that a sample exhibits *G0S2* expression concurrent with high *G0S2* CpG island methylation, *G0S2* methylation is prioritized to assign the sample to COC3. To determine the cutoff for ΔC_{T} *BUB1B -* ΔC_{T} *PINK1* to define COC1, clinical outcomes data is used to establish a threshold indicative of a COC score. Non-COC3 patients with good outcomes are identified as those with no evidence of recurrence or disease-associated events within at least two years of clinical observation after complete R0 resection and no evidence of metastasis at any time point. Non-COC3 patients with poor outcomes are identified as those with metastatic disease at any time point (including diagnosis) or those with recurrence or disease-associated events within two years of complete R0 resection. Using the calculated ΔC_{T} *BUB1B -* ΔC_{T} *PINK1* value for each non-COC3 patient sample combined with the binary assignment to "good" or "poor" outcomes, ROC curve analysis is performed to determine the appropriate threshold or ΔC_{T} *BUB1B -* ΔC_{T} *PINK1* above which defines COC1. Non-COC3 Patients with a score above the threshold are assigned to COC1. All non-COC3 samples that have ΔC_{T} *BUB1B* - ΔC_{T} *PINK1* below the threshold are assigned to COC2.

## Claims

1. A method for characterizing adrenocortical carcinoma (ACC), comprising:
a) contacting a sample from a subject diagnosed with ACC with reagents for determining the level of expression of at least *BUB1B, PINK1,* and *GOS2* and the methylation status of *GOS2;* and
b) characterizing said ACC as molecular subgroup COC1, COC2, or COC3 based on said level of expression of *BUB1B, PINK1,* and *GOS2* and methylation status of *GOS2,* wherein said characterizing comprises determining a *BUB1B-PINK1* expression score, and wherein
i) a *BUB1B-PINK1* expression score above a threshold level is indicative of COC1;
ii) *a BUB1B-PINK1* expression score below a threshold level and less than 5% *GOS2* methylation is indicative of COC2;
iii) *a BUB1B-PINK1* expression score below a threshold level and greater than 5% *GOS2* methylation is indicative of COC3.

2. The method of claim 1, wherein said characterizing further comprises determining a prognosis, wherein said prognosis is preferably likelihood of metastasis, wherein further preferably COC3 classification is indicative of metastasis of said ACC and an aggressive cancer.

3. The method of claim 1 or 2, wherein said method further comprises determining a treatment course of action based on said molecular subgroup.

4. The method of claim 3, wherein said treatment course of action comprises adjuvant cytotoxic chemotherapy in subjects with COC3 tumors.

5. The method of any of claims 1-4, wherein said biological sample is selected from the group consisting of a tissue sample, a biopsy sample, a blood sample, and a urine sample.

6. The method of any one of claims 1 to 5, wherein said reagents are selected from the group consisting of a nucleic acid probe or probes that hybridizes to *BUB1B, PINK1,* and *GOS2,* one or more nucleic acid primers for the amplification or extension of *BUB1B, PINK1,* and *GOS2,* and one or more nucleic acid primers that bind specifically to methylated *GOS2* nucleic acids.

7. An adjuvant cytotoxic chemotherapy agent for use in a method of treating ACC in a subject with indication of COC3 tumors, said method comprising:
a) contacting a sample from a subject diagnosed with ACC with reagents for determining the level of expression of *BUB1B, PINK1,* and *GOS2* and the methylation status of *GOS2;*
b) characterizing said ACC as molecular subgroup COC3 based on said level of expression of *BUB1B, PINK1,* and *GOS2* and methylation status of *G0S2,* wherein said characterizing comprises determining a *BUB1B-PINK1* expression score and wherein a determined *BUB1B-PINK1* expression score below a threshold level and greater than 5% *GOS2* methylation is indicative of COC3 tumors.

8. The adjuvant cytotoxic chemotherapy agent for use in a method of claim 7, wherein said biological sample is selected from the group consisting of a tissue sample, a biopsy sample, a blood sample, and a urine sample.

9. The adjuvant cytotoxic chemotherapy agent for use in a method of claim 7 or 8, wherein said reagents are selected from the group consisting of a nucleic acid probe or probes that hybridizes to *BUB1B, PINK1,* and *GOS2,* one or more nucleic acid primers for the amplification or extension of *BUB1B, PINK1,* and *GOS2,* and one or more nucleic acid primers that bind specifically to methylated *GOS2* nucleic acids.

10. A method of assaying gene expression and methylation, comprising:
a) contacting a sample from a subject diagnosed with ACC with reagents for determining the level of expression of *BUB1B, PINK1,* and *GOS2* and the methylation status of *GOS2;* and
b) identifying said level of expression of *BUB1B, PINK1,* and *GOS2* and methylation status of *GOS2,* wherein said identifying comprises determining a *BUB1B-PINK1* expression score and wherein
i) a *BUB1B-PINK1* expression score above a threshold level is indicative of COC1;
ii) *a BUB1B-PINK1* expression score below a threshold level and less than 5% *GOS2* methylation is indicative of COC2;
iii) *a BUB1B-PINK1* expression score below a threshold level and greater than 5% *GOS2* methylation is indicative of COC3.

11. The method of claim 10, wherein said biological sample is selected from the group consisting of a tissue sample, a biopsy sample, a blood sample, and a urine sample.

12. The method of claim 10 or 11, wherein said reagents are selected from the group consisting of a nucleic acid probe or probes that hybridizes to *BUB1B, PINK1,* and *GOS2,* one or more nucleic acid primers for the amplification or extension of *BUB1B, PINK1,* and *GOS2,* and one or more nucleic acid primers that bind specifically to methylated *GOS2* nucleic acids.

13. Use of a kit or system in a method of any of the preceding claims, said kit or system comprising:
reagents for determining the level of expression of *BUB1B, PINK1,* and *GOS2* and the methylation status of *G0S2,* wherein said reagents are selected from the group consisting of a nucleic acid probe or probes that hybridizes to *BUB1B, PINK1,* and *GOS2,* one or more nucleic acid primers for the amplification or extension of *BUB1B, PINK1,* and *GOS2,* and one or more nucleic acid primers that bind specifically to methylated *GOS2* nucleic acids.

14. The use of a kit or system of claim 13, wherein said kit or system further comprises reagents for detection of methylated DNA, wherein said reagents preferably comprise bisulfite.

## Patentansprüche

1. Verfahren zur Charakterisierung eines adrenokortikalen Karzinoms (ACC), umfassend:
a) In-Kontakt-Bringen einer Probe von einem Patienten, bei dem ACC diagnostiziert wurde, mit Reagenzien zur Bestimmung des Expressionsniveaus von mindestens *BUB1B, PINK1* und *G0S2* und des Methylierungsstatus von G0S2; und
b) Charakterisieren des ACC als molekulare Untergruppe COC1, COC2 oder COC3 auf der Grundlage des Expressionsniveaus von *BUB1B, PINK1* und *G0S2* und des Methylierungsstatus von *G0S2,* wobei das Charakterisieren das Bestimmen eines *BUB1B-PINK1* Expressionswerts umfasst, und wobei
i) ein *BUB1B-PINK1* Expressionswert über einem Schwellenwert auf COC1 hinweist;
ii) ein *BUB1B-PINK1* Expressionswert unterhalb eines Schwellenwerts und weniger als 5% *G0S2*-Methylierung auf COC2 hinweist;
iii) ein *BUB1B-PINK1* Expressionswert unterhalb eines Schwellenwerts und mehr als 5% *G0S2*-Methylierung auf COC3 hinweist.

2. Verfahren nach Anspruch 1, wobei die Charakterisierung des Weiteren die Bestimmung einer Prognose umfasst, wobei die Prognose vorzugsweise die Wahrscheinlichkeit einer Metastasierung ist, wobei weiter vorzugsweise die COC3-Klassifizierung auf eine Metastasierung des ACC und einen aggressiven Krebs hinweist.

3. Verfahren nach Anspruch 1 oder 2, wobei das Verfahren des Weiteren die Bestimmung einer Behandlungsvorgehensweise auf der Grundlage der molekularen Untergruppe umfasst.

4. Verfahren nach Anspruch 3, wobei die Behandlungsvorgehensweise eine adjuvante zytotoxische Chemotherapie bei Patienten mit COC3-Tumoren umfasst.

5. Verfahren nach einem der Ansprüche 1 bis 4, wobei die biologische Probe aus der Gruppe ausgewählt ist, die aus einer Gewebeprobe, einer Biopsieprobe, einer Blutprobe und einer Urinprobe besteht.

6. Verfahren nach einem der Ansprüche 1 bis 5, wobei die Reagenzien aus der Gruppe ausgewählt sind, bestehend aus einer Nukleinsäuresonde oder - sonden, die an *BUB1B, PINK1* und *G0S2* hybridisiert, einem oder mehreren Nukleinsäureprimern zur Amplifikation oder Verlängerung von *BUB1B, PINK1* und *G0S2,* und einen oder mehrere Nukleinsäureprimern, die spezifisch an methylierte *GOS2*-Nukleinsäuren binden.

7. Adjuvantes zytotoxisches Chemotherapeutikum zur Verwendung in einem Verfahren zur Behandlung von ACC bei einem Patienten mit Anzeichen von COC3-Tumoren, wobei das Verfahren umfasst:
a) In-Kontakt-Bringen einer Probe von einem Patienten, bei dem ACC diagnostiziert wurde, mit Reagenzien zur Bestimmung des Expressionsniveaus von *BUB1B, PINK1* und *G0S2* und des Methylierungsstatus von G0S2;
b) Charakterisieren des ACC als molekulare Untergruppe COC3 basierend auf dem Expressionsniveau von *BUB1B, PINK1* und *G0S2* und dem Methylierungsstatus von *G0S2,* wobei das Charakterisieren das Bestimmen eines BUB1B-PINK1-Expressionswerts umfasst und wobei ein bestimmter BUB1B-PINK1-Expressionswert unterhalb eines Schwellenwerts und größer als 5% *G0S2*-Methylierung auf COC3-Tumore hinweist.

8. Adjuvantes zytotoxisches Chemotherapeutikum zur Verwendung in einem Verfahren nach Anspruch 7, wobei die biologische Probe aus der Gruppe ausgewählt ist, die aus einer Gewebeprobe, einer Biopsieprobe, einer Blutprobe und einer Urinprobe besteht.

9. Adjuvantes zytotoxisches Chemotherapeutikum zur Verwendung in einem Verfahren nach Anspruch 7 oder 8, wobei die Reagenzien aus der Gruppe ausgewählt sind, bestehend aus einer Nukleinsäuresonde oder -sonden, die an *BUB1B, PINK1* und *G0S2* hybridisieren, einem oder mehreren Nukleinsäureprimern zur Amplifikation oder Verlängerung von *BUB1B, PINK1* und *G0S2* und einem oder mehreren Nukleinsäureprimern, die spezifisch an methylierte *GOS2*-Nukleinsäuren binden.

10. Verfahren zur Untersuchung der Genexpression und -methylierung, umfassend
a) In-Kontakt-Bringen einer Probe von einem Patienten, bei dem ACC diagnostiziert wurde, mit Reagenzien zur Bestimmung des Expressionsniveaus von *BUB1B, PINK1* und *G0S2* und des Methylierungsstatus von G0S2; und
b) Identifizieren des Expressionsniveaus von *BUB1B, PINK1* und *G0S2* und des Methylierungsstatus von *G0S2,* wobei das Identifizieren das Bestimmen eines BUB1B-PINK1-Expressionswerts umfasst und wobei
i) ein BUB1B-PINK1-Expressionswert über einem Schwellenwert auf COC1 hinweist;
ii) ein BUB1B-PINK1-Expressionswert unter einem Schwellenwert und weniger als 5% *G0S2*-Methylierung auf COC2 hinweist;
iii) ein BUB1B-PINK1-Expressionswert unter einem Schwellenwert und mehr als 5% *G0S2*-Methylierung auf COC3 hinweist.

11. Verfahren nach Anspruch 10, wobei die biologische Probe aus der Gruppe ausgewählt ist, die aus einer Gewebeprobe, einer Biopsieprobe, einer Blutprobe und einer Urinprobe besteht.

12. Verfahren nach Anspruch 10 oder 11, wobei die Reagenzien aus der Gruppe ausgewählt sind, bestehend aus einer Nukleinsäuresonde oder -sonden, die an *BUB1B, PINK1* und *G0S2* hybridisiert, einem oder mehreren Nukleinsäureprimern zur Amplifikation oder Verlängerung von *BUB1B, PINK1* und *G0S2* und einem oder mehreren Nukleinsäureprimern, die spezifisch an methylierte *GOS2*-Nukleinsäuren binden.

13. Verwendung eines Kits oder Systems in einem Verfahren nach einem der vorhergehenden Ansprüche, wobei das Kit oder System Folgendes umfasst: Reagenzien zur Bestimmung des Expressionsniveaus von *BUB1B, PINK1* und *G0S2* und des Methylierungsstatus von *G0S2,* wobei die Reagenzien aus der Gruppe ausgewählt sind, bestend aus einer Nukleinsäuresonde oder -sonden, die an *BUB1B, PINK1* und *G0S2* hybridisiert, einem oder mehreren Nukleinsäureprimern zur Amplifikation oder Verlängerung von *BUB1B, PINK1* und *G0S2,* und einen oder mehrere Nukleinsäureprimer, die spezifisch an methylierte *GOS2*-Nukleinsäuren binden.

14. Verwendung eines Kits oder Systems nach Anspruch 13, wobei das Kit oder System weiter Reagenzien zum Nachweis von methylierter DNA umfasst, wobei die Reagenzien vorzugsweise Bisulfit umfassen.

## Revendications

1. Procédé de caractérisation de carcinome corticosurrénalien (ACC), comprenant :
a) la mise en contact d'un échantillon provenant d'un sujet diagnostiqué d'ACC avec des réactifs pour déterminer le niveau d'expression d'au moins *BUB1B, PINK1,* et *G0S2* et l'état de méthylation de *G0S2 ;* et
b) la caractérisation dudit ACC comme sous-groupe moléculaire COC1, COC2, ou COC3 sur la base dudit niveau d'expression de *BUB1B, PINK1,* et *G0S2* et de l'état de méthylation de *G0S2,* dans lequel ladite caractérisation comprend la détermination d'un score d'expression de *BUB1B-PINK1,* et dans lequel
i) un score d'expression de *BUB1B-PINK1* supérieur à un niveau seuil est indicatif de COC1 ;
ii) un score d'expression de *BUB1B-PINK1* inférieur à un niveau seuil et inférieur à 5 % de méthylation de *G0S2* est indicatif de COC2 ;
iii) un score d'expression de *BUB1B-PINK1* inférieur à un niveau seuil et supérieur à 5 % de méthylation de *G0S2* est indicatif de COC3.

2. Procédé selon la revendication 1, dans lequel ladite caractérisation comprend en outre la détermination d'un pronostic, dans lequel ledit pronostic est de préférence une probabilité de métastase, dans lequel en outre, de préférence, la classification COC3 est indicative d'une métastase dudit ACC et d'un cancer agressif.

3. Procédé selon la revendication 1 ou 2, dans lequel ledit procédé comprend en outre la détermination d'un cours d'action de traitement basé sur ledit sous-groupe moléculaire.

4. Procédé selon la revendication 3, dans lequel ledit plan d'action de traitement comprend une chimiothérapie cytotoxique adjuvante chez des sujets atteints de tumeurs COC3.

5. Procédé selon l'une quelconque des revendications 1 à 4, dans lequel ledit échantillon biologique est choisi dans le groupe constitué par un échantillon de tissu, un échantillon de biopsie, un échantillon de sang, et un échantillon d'urine.

6. Procédé selon l'une quelconque des revendications 1 à 5, dans lequel lesdits réactifs sont choisis dans le groupe constitué par une sonde ou des sondes d'acide nucléique qui s'hybrident à *BUB1B, PINK1,* et *G0S2,* une ou plusieurs amorces d'acide nucléique pour l'amplification ou l'extension de *BUB1B, PINK1,* et *G0S2,* et une ou plusieurs amorces d'acide nucléique qui se lient spécifiquement à des acides nucléiques méthylés de *G0S2.*

7. Agent chimiothérapeutique cytotoxique adjuvant destiné à être utilisé dans un procédé de traitement d'ACC chez un sujet avec indication de tumeurs COC3, ledit procédé comprenant :
a) la mise en contact d'un échantillon provenant d'un sujet diagnostiqué d'ACC avec des réactifs pour déterminer le niveau d'expression de *BUB1B, PINK1,* et *G0S2* et l'état de méthylation de *G0S2 ;*
b) la caractérisation dudit ACC en tant que sous-groupe moléculaire COC3 sur la base dudit niveau d'expression de *BUB1B, PINK1,* et *G0S2* et de l'état de méthylation de *G0S2,* dans lequel ladite caractérisation comprend la détermination d'un score d'expression de *BUB1B-PINK1* et dans lequel un score d'expression de *BUB1B-PINK1* déterminé inférieur à un niveau seuil et supérieur à 5 % de méthylation de *G0S2* est indicatif de tumeurs COC3.

8. Agent chimiothérapeutique cytotoxique adjuvant destiné à être utilisé dans un procédé selon la revendication 7, dans lequel ledit échantillon biologique est choisi dans le groupe constitué par un échantillon de tissu, un échantillon de biopsie, un échantillon de sang, et un échantillon d'urine.

9. Agent chimiothérapeutique cytotoxique adjuvant destiné à être utilisé dans un procédé selon la revendication 7 ou 8, dans lequel lesdits réactifs sont choisis dans le groupe constitué par une sonde ou des sondes d'acide nucléique qui s'hybrident à *BUB1B, PINK1,* et *G0S2,* une ou plusieurs amorces d'acide nucléique pour l'amplification ou l'extension de *BUB1B, PINK1,* et *G0S2,* et une ou plusieurs amorces d'acide nucléique qui se lient spécifiquement à des acides nucléiques méthylés de *G0S2.*

10. Procédé de dosage d'expression génique et de méthylation, comprenant :
a) la mise en contact d'un échantillon provenant d'un sujet diagnostiqué d'ACC avec des réactifs pour déterminer le niveau d'expression de *BUB1B, PINK1,* et *G0S2* et l'état de méthylation de *G0S2 ;* et
b) l'identification dudit niveau d'expression de *BUB1B, PINK1,* et *G0S2* et de l'état de méthylation de *G0S2,* dans lequel ladite identification comprend la détermination d'un score d'expression de *BUB1B-PINK1* et dans lequel
i) un score d'expression de *BUB1B-PINK1* supérieur à un niveau seuil est indicatif de COC1 ;
ii) un score d'expression de *BUB1B-PINK1* inférieur à un niveau seuil et inférieur à 5 % de méthylation de *G0S2* est indicatif de COC2 ;
iii) un score d'expression de *BUB1B-PINK1* inférieur à un niveau seuil et supérieur à 5 % de méthylation de *G0S2* est indicatif de COC3.

11. Procédé selon la revendication 10, dans lequel ledit échantillon biologique est choisi dans le groupe constitué par un échantillon de tissu, un échantillon de biopsie, un échantillon de sang, et un échantillon d'urine.

12. Procédé selon la revendication 10 ou 11, dans lequel lesdits réactifs sont choisis dans le groupe constitué par une sonde ou des sondes d'acide nucléique qui s'hybrident à *BUB1B, PINK1,* et *G0S2,* une ou plusieurs amorces d'acide nucléique pour l'amplification ou l'extension de *BUB1B, PINK1,* et *G0S2,* et une ou plusieurs amorces d'acide nucléique qui se lient spécifiquement à des acides nucléiques méthylés de *G0S2.*

13. Utilisation d'un kit ou d'un système dans un procédé selon l'une quelconque des revendications précédentes, ledit kit ou système comprenant :
des réactifs pour déterminer le niveau d'expression de *BUB1B, PINK1,* et *G0S2* et l'état de méthylation de *G0S2,* dans laquelle lesdits réactifs sont choisis dans le groupe constitué par une sonde ou des sondes d'acide nucléique qui s'hybrident à *BUB1B, PINK1,* et *G0S2,* une ou plusieurs amorces d'acide nucléique pour l'amplification ou l'extension de *BUB1B, PINK1,* et *G0S2,* et une ou plusieurs amorces d'acide nucléique qui se lient spécifiquement à des acides nucléiques méthylés de *G0S2.*

14. Utilisation d'un kit ou système selon la revendication 13, dans laquelle ledit kit ou système comprend en outre des réactifs pour la détection d'ADN méthylé, dans laquelle lesdits réactifs comprennent de préférence du bisulfite.
